# EUROPEAN PATENT SPECIFICATION

(11) **EP 2 311 504 B1**
(45) Date of publication and mention of the grant of the patent: **05.12.2018**
(21) Application number: 10186489.0
(22) Date of filing: 05.10.2010
(51) Int. Cl.: A61L 15/44, A61L 15/60, A61L 15/46

(54) **Wound covering comprising octenidine dihydrochloride for use in the antisepsis of catheter insertion points**
Wundenabdeckung mit Octenidin-Dihydrochlorid zur antiseptischen Verwendung von Kathetereinführstellen
Couverture des plaies comportant du dihydrochlorure d'octénidine pour l'antisepsie de points d'insertion de cathéter

(30) Priority: 15.10.2009 DE 102009049506
(43) Date of publication of application: 20.04.2011
(60) Divisional application: 16196419.2
(73) Proprietor: L'Air Liquide Société Anonyme pour l'Etude et l'Exploitation des Procédés Georges Claude, 75007 Paris (FR); SCHÜLKE & MAYR GMBH, 22851 Norderstedt (DE)
(72) Inventor: Behrends, Sabine, 25482 Appen (DE); Baur, Boris, 22307 Hamburg (DE); Dettmann, Andreas, 22157 Hamburg (DE)
(74) Representative: Conan, Philippe Claude

(56) References cited:
- DE-A1-102006 001 954
- US-A1- 2009 187 130
- M. DETTENKOFER ET AL: "Effect of Skin Disinfection with Octenidine Dihydrochloride on Insertion Site Colonization of Intravascular Catheters", INFECTION, vol. 30, no. 5, 1 October 2002 (2002-10-01), pages 282-285, XP055129167, ISSN: 0300-8126, DOI: 10.1007/s15010-002-2182-2
- ANDREAS TIETZ ET AL: "Octenidine Hydrochloride for the Care of Central Venous Catheter Insertion Sites in Severely Immunocompromised Patients -", INFECTION CONTROL AND HOSPITAL EPIDEMIOLOGY, vol. 26, no. 8, 1 August 2005 (2005-08-01) , pages 703-707, XP055129168, ISSN: 0899-823X, DOI: 10.1086/502606

## Description

The invention relates to a wound covering for use in the antisepsis of catheter insertion points. The wound covering comprises a transparent film and, applied to the film, a transparent hydrogel which comprises octenidine dihydrochloride. It also relates to a wound covering set.

To prevent catheter-associated infections, it is usually customary to disinfect and to thereby care for the skin prior to positioning the catheter, to position the catheter and then to cover the catheter insertion point with a customary wound dressing. Alternatively, the insertion point can be covered with a wound dressing which is antimicrobially finished. One example of an antimicrobially finished wound covering is the commercial product biopatch®, which is supplied by Johnson & Johnson and comprises chlorhexidine gluconate as antimicrobial active ingredient. Another known wound covering is the product SilvaSorb Site® from Medline which is antimicrobially finished with silver.

Tietz et al. (Infect Control Hosp Epidemiol. 2005, August, 26 (8): 703-7) describe the use of octenidine dihydrochloride for the care of venous catheter insertion points in patients with a severely weakened immune system.

However, it has been found that when using a wound dressing and also when using a wound covering, on account of the lack of transparency of the carrier material, catheter insertion points are difficult to assess visually and there is therefore the risk that inflammation arises that has become protracted due to it being unnoticed. Added to this is the high rate of incompatibility reactions when using chlorhexidine gluconate (especially in sensitive patients, such as e.g. patients with weakened immune system, oncologic patients) or silver as active ingredient. Furthermore, the specified wound coverings have to be covered with an additional (adhesive) dressing or a film in order to ensure the greatest possible freedom from germs of the catheter insertion point. Added to this, for both of the described methods in the prior art, is an inadequate antimicrobial effect over a prolonged period, which makes it necessary, but undesirable, to frequently change the wound covering/the wound dressing in order to be able to reliably exclude an infection. Such a change is always associated with the risk of a new infection of the catheter insertion point. On account of these disadvantages, a tending of catheter insertion points that avoids infections has hitherto not been satisfactorily possible.

German patent application DE 10 2006 001 954 A1 discloses an antiseptic wound dressing comprising an alginate and octenidine dihydrochloride.

U.S. patent application publication US 2009/0187130 A1 discloses an island dressing comprising an adhesive hydrogel island pad comprising an hydrogel composition comprising less than 45 weight percent water, from about 10 to about 50 weight percent swellable crosslinked poly(N-vinyl lactam), from about 50 to 90 weight percent of a swelling agent and a modifying polymer in a ratio modifying polymer to swelling agent of 1:9 and up to 10 weight percent of an antimicrobial agent.

The object of the present invention was therefore to provide means for preventing catheter-associated infections, with the help of which the described problems of the prior art can be avoided.

Surprisingly, it has now been found that this object is achieved by a wound covering according to Claim 1.

The scope of the invention is defined by the claims. Any references in the description to methods of treatment refer to the compounds, pharmaceutical compositions and medicaments of the present invention for use in a method for treatment of the human (or animal) body by therapy (or for diagnosis).

The invention is based inter alia on the fact that it has been found that hydrogels release the antimicrobial active ingredient octenidine dihydrochloride not only quickly, but surprisingly also in a long-lasting manner. This avoids the incompatibility reactions which are observed with chlorhexidine gluconate or silver as active ingredient. In addition, infections are reliably and long-lastingly prevented. Consequently, in the field of skin antisepsis at an insertion point, it is possible e.g. to bring about increased performance and also an increase in safety compared with customary skin antisepsis. Moreover, the transparent film and the transparent hydrogel permit good monitoring of the catheter insertion point, as a result of which it is possible to avoid an inflammation thereof that has become protracted due to it being unnoticed without a frequent change of the wound covering being necessary for checking.

### I. Wound covering

### a) Film

The wound covering according to the invention has a transparent film. The film does not have to be completely transparent. For example, it suffices if the part of the film covering the catheter insertion point upon subsequent use is transparent. However, the wound covering is preferably transparent not only in the area which has the hydrogel, but also in the adhesive area and especially over the entire area.

The transparency or permeability to light is measured in accordance with ASTM-D 1003-77. According to this, the film can be an opaque to translucent film, but preferably a transparent film. Within the context of the present invention, "transparent film" means a film, the permeability to light of which in accordance with ASTM-D 1003-77 is at least 90%, preferably at least 95%.

The film used according to the invention can be formed as one or more layers, but is preferably formed as one layer. Here, in all embodiments of the invention, preference is given to rectangular films. Typical dimensions of the film are about 3 x about 3 cm. Alternatively, it is possible to design the films according to the invention round in shape; the diameter is then preferably about 3 cm.

A typical thickness of the film used according to the invention is 5-500 µm, preferably 50-200 µm.

Suitable film materials are polymer films, preferably with breathable properties, e.g. polyester polyurethanes or polyether polyurethanes (such as e.g. Estane®, B.F. Goodridge), polyether polyamides (such as e.g. PEBAX®, Arkema), but also hydrophilic and hydrophobic polyurethanes or those with elastomeric properties (such as e.g. HYTREL®, DuPont).

The film has a particularly good water vapour permeability of > 500 g/m²/24 h, but especially of > 2500 g/m²/24 h. The water vapour permeability is determined in accordance with DIN EN 13726-2: 2002.

In this connection, one embodiment is possible in which the film of the wound covering is coated in one area with an adhesive, for example based on poly(meth)acrylate, and is provided in another area with the hydrogel. The area finished with the hydrogel constitutes preferably 5 to 95%, preferably 50 to 80%, such as about 70%, of the total area of the wound covering.

The wound covering according to the invention has a peelable film particularly preferably on the side of the film which has the hydrogel. Peelable films of this type are known from the prior art. For example, peelable films made of polyethylene terephthalalate, polyethylene, polypropylene or polyvinyl chloride are used. This thus gives rise, in the areas of the wound covering which have the hydrogel, to a layer sequence film-hydrogel-peelable film, and in the areas which do not have hydrogel, to a layer sequence film-peelable film.

### b) Hydrogel

The hydrogel used according to the invention is transparent and skin-friendly. The transparency or permeability to light is measured in accordance with ASTM-D 1003-77. The hydrogel is a transparent gel. Within the context of the present invention, "transparent gel" means a gel, the permeability to light of which in accordance with ASTM-D 1003-77 is at least 90%, preferably at least 95%.

Preferably, the hydrogel is sterile. In one preferred embodiment, it consists of the components b1) to b4).

The hydrogel is applied to the film in a thickness of 0.1-5 mm.

### b1) Octenidine dihydrochloride

Octenidine dihydrochloride corresponds to the following formula:

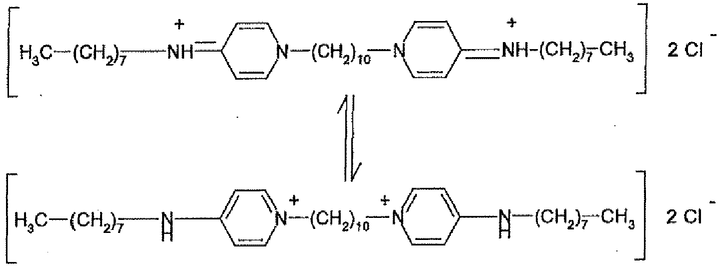

The active ingredient is used successfully by Schülke & Mayr GmbH, Norderstedt, Germany inter alia in the commercial products Octenisept® (wound and mucosa antisepsis), Octeniderm® (preoperative skin antiseptic), Octenidol® (antimicrobial mouth rinse solution) and Octenisan® (antimicrobial washing lotion). Octenidine dihydrochloride has a wide antimicrobial spectrum of activity. According to the invention, it has been found that the active ingredient octenidine dihydrochloride is particularly advantageously released from a hydrogel, as demonstrated by the examples. The amount of octenidine dihydrochloride in the hydrogel is 0.005 to 0.5% by weight, such as 0.01 to 0.2% by weight and in particular 0.05 to 0.1% by weight.

### b2) Thickener

Thickener is present in the hydrogel in an amount of from 1.5 to 4% by weight and in particular 2 to 3% by weight.

Preferred thickeners are hydroxyethylcellulose.

Particularly preferred hydrogels according to the invention and their preparation are given below:

### Hydrogel I (data in percent by weight)

0.05% octenidine dihydrochloride
9.90%propylene glycol
2.50% hydroxyethylcellulose 4000
87.55% demineralized (dem.) water

This exemplary hydrogel is prepared as follows: water is introduced as initial charge and propylene glycol and octenidine dihydrochloride are dissolved therein with heating to 60°C to give a clear solution. The hydroxyethylcellulose is then added and stirring is carried out until the distribution is homogeneous. The product is then left to swell overnight.

### Hydrogel II (data in percent by weight)

0.10% octenidine
2.85% glycerol 85%
94.55% dem. water
2.50% hydroxyethylcellulose 4000.

The viscosity of the hydrogels used according to the invention is 10 000-3000 mPa*s, measured by measurement, corresponding to DIN 53019, of viscosities and flow curves using rotary viscometers.

The pH of the hydrogels used according to the invention is generally in the pH range from 2 to 10, preferably in the pH range from 3 to 8, particularly preferably in the pH range from 4 to 7.

The wound covering according to the invention can be used, for example, for covering central venous catheters (CVC) and for covering other peripheral accesses. Advantages of the use according to the invention of a hydrogel comprising octenidine dihydrochloride are:
- broad and rapid-onset microbicidal effectiveness
- long-lasting microbicidal effectiveness
- very good to adequately good release of the active ingredient from the hydrogel
- very good stability of the active ingredient and of the hydrogel
- very good thermal stability of the active ingredient and of the hydrogel
- heat-sterilizable
- clear and/or translucent and/or transparent, even after prolonged storage under unfavourable storage conditions
- good adhesion to skin, mucosa and medical articles such as catheters
- good spreadability on surfaces
- very good and rapid wetting of surfaces
- very good skin compatibility, very good compatibility on injured skin, very slight cytotoxic effect
- pleasant skin feel, possibly skin-cooling effect
- free from undesired by-products or degradation products
- can be washed off easily, can be removed without residues
- material-conserving
- economical
- high acceptance among medical personnel and patients.

The advantages of the invention arise in particular from the following examples.

### Examples

### Method:

### Bioavailability of octenidine dihydrochloride in a hydrogel

The bioavailability of octenidine dihydrochloride from a hydrogel was tested using the Sartorius liberation model (type SM 16755).

The ointment liberation cell consists of three see-through Plexiglas plates held together by bolts and nuts. Chambers are built into two plates. Heating water passes through the heating space in the middle plate. In the same plate, there is, opposite the heating space, the ointment chamber for accommodating the substance under investigation. In the plate, the acceptor medium flows through the ripple plate, where it comes into contact with the membrane of the released active ingredient.

Temperature medium and acceptor medium thus have two independent cycles. The sample for determining the content is drawn from the acceptor medium chamber.
Test product: Hydrogel I
Photometer method: Photometer Cadas 100, Dr. Lange (280 nm)

A calibration for the active ingredient octenidine dihydrochloride is firstly carried out.

The test product is weighed into the ointment chamber. Samples are taken at the specified times. The removed sample volume is replaced completely by new acceptor medium. At the end of the total runtime, the samples are measured at 280 nm in 1 cm quartz cuvettes.

The results are shown in the table below:

| Time (h) | Released amounts of octenidine dihydrochloride (in % of the total amount) |
|---|---|
| 0.5 | 10.83 |
| 1 | 16.73 |
| 2 | 27.54 |
| 3 | 36.22 |
| 4 | 43.15 |
| 5 | 50.27 |
| 6 | 56.06 |
| 8 | 65.31 |
| 24 | 90.04 |

The results show that a good and continuous release of the active ingredient octenidine dihydrochloride from the hydrogel takes place. After 24 hours, 90% of the contained active ingredient have been released from the gel.

## Claims

1. Wound covering comprising:
a) a transparent film having a permeability to light measured in accordance with ASTM-D 1003-77 of at least 90%, and a water vapour permeability > 500g/m²/24h determined in accordance with DIN EN 13726-2: 2002 and
b) applied to the film in a thickness of 0.1 to 5mm, a transparent hydrogel having a permeability to light measured in accordance with ASTM-D 1003-77 of at least 90%, which consists of :
b1) 0.001 to 1% by weight octenidine dihydrochloride,
b2) 0.5 to 10% by weight thickener selected from xanthan gums, thickening silicas, polyvinyl alcohols, polyacrylates, gelatin, pectin, casein, agar agar, starch, tragacanth, polyvinylpyrrolidone, cellulose and cellulose derivatives and mixtures thereof,
b3) 0.1 to 20% by weight of polyol selected from propylene glycol and/or glycerol and
b4) water; said hydrogel having a viscosity between 15000 and 1000 mpas measured by measurement corresponding to DIN 53019.

2. Wound covering according to Claim 1, **characterized in that** the hydrogel consists of:
0.05% by weight octenidine dihydrochloride,
9.90% by weight propylene glycol,
2.50% by weight hydroxyethylcellulose 4000,
87.55% by weight demineralized water.

3. Wound covering according to Claim 1, **characterized in that**, the hydrogel consists of:
0.10% by weight octenidine dihydrochloride,
2.85% by weight glycerol,
2.50% by weight hydroxyethylcellulose 4000,
94.55% by weight demineralized water.

## Patentansprüche

1. Wundabdeckung, umfassend:
a) einen transparenten Film, der eine gemäß ASTM-D 1003-77 gemessene Licht-Permeabilität von mindestens 90 % und eine gemäß DIN EN 13726-2: 2002 bestimmte Wasserdampf-Permeabilität von > 500 g/m²/24h aufweist und
b) ein mit einer Dicke von 0,1 bis 5 mm auf den Film aufgetragenes, transparentes Hydrogel, das eine gemäß ASTM-D 1003-77 gemessene Licht-Permeabilität von mindestens 90 % aufweist, welches besteht aus:
b1) 0,001 bis 1 Gew.-% Octenidindihydrochlorid,
b2) 0,5 bis 10 Gew.-% Verdickungsmittel ausgewählt aus Xanthangummis, verdickenden Kieselsäuren, Polyvinylalkoholen, Polyacrylaten, Gelatine, Pektin, Casein, Agar-Agar, Stärke, Tragacanth, Polyvinylpyrrolidon, Cellulose und Cellulosederivaten und Mischungen davon,
b3) 0,1 bis 20 Gew.-% eines Polyols ausgewählt aus Propylenglkykol und/oder Gylcerin und
b4) Wasser;
wobei das Hydrogel eine Viskosität gemessen durch Messung entsprechend DIN 53019 zwischen 15.000 und 1000 mPas aufweist.

2. Wundabdeckung nach Anspruch 1, **dadurch gekennzeichnet, dass** das Hydrogel besteht aus:
0,05 Gew.-% Octenidindihydrochlorid,
9,90 Gew.-% Propylenglykol,
2,50 Gew.-% Hydroxyethylcellulose 4000,
87,55 Gew.-% demineralisiertem Wasser.

3. Wundabdeckung nach Anspruch 1, **dadurch gekennzeichnet, dass** das Hydrogel besteht aus:
0,10 Gew.-% Octenidindihydrochlorid,
2,85 Gew.-% Glycerin,
2,50 Gew.-% Hydroxyethylcellulose 4000,
94,55 Gew.-% demineralisiertem Wasser.

## Revendications

1. Revêtement de plaie comprenant :
a) un film transparent ayant une perméabilité à la lumière mesurée selon ASTM-D 1003-77 d'au moins 90 % et une perméabilité à la vapeur d'eau > 500 g/m²/24 h déterminée selon la norme DIN EN 13726-2:2002 et
b) appliqué au film en une épaisseur de 0,1 à 5 mm, un hydrogel transparent ayant une perméabilité à la lumière mesurée selon ASTM-D 1003-77 d'au moins 90 %, qui consiste en :
b1) 0,001 à 1 % en poids de dihydrochlorure d'octénidine,
b2) 0,5 à 10 % en poids d'un épaississant sélectionné parmi des gommes de xanthane, des silices épaississantes, des alcools polyvinyliques, des polyacrylates, de la gélatine, de la pectine, de la caséine, de l'agar-agar, de l'amidon, de l'adragante, de la polyvinylpyrrolidone, de la cellulose et des dérivés de cellulose et des mélanges de ceux-ci,
b3) 0,1 à 20 % en poids de polyol sélectionné parmi du propylène glycol et/ou du glycérol, et
b4) de l'eau ;
ledit hydrogel ayant une viscosité entre 15 000 et 1 000 mpas mesurée par une mesure correspondant à la norme DIN 53019.

2. Revêtement de plaie selon la revendication 1, **caractérisé en ce que** l'hydrogel consiste en :
0,05 % en poids de dihydrochlorure d'octénidine,
9,90 % en poids de propylène glycol,
2,50 % en poids d'hydroxyéthylcellulose 4000,
87,55 % en poids d'eau déminéralisée.

3. Revêtement de plaie selon la revendication 1, **caractérisé en ce que** l'hydrogel consiste en :
0,10 % en poids de dihydrochlorure d'octénidine,
2,85 % en poids de glycérol,
2,50 % en poids d'hydroxyéthylcellulose 4000,
94,55 % en poids d'eau déminéralisée.
